# DEMANDE DE BREVET EUROPEEN

(11) **EP 0 930 516 A1**
(43) Date de publication de la demande: **21.07.1999**
(21) Numéro de dépôt: 99400087.5
(22) Date de dépôt: 14.01.1999
(51) Int. Cl.: G01T 1/164

(54) **Articulation orientable monté sur une caméra de scintigraphie pour dispositif de correction d'atténuation de transmission**

(30) Priorité: 16.01.1998 FR 9800448
(71) Demandeur: SMV INTERNATIONAL, 78530 Buc Cedex (FR)
(72) Inventeur: Geay, Jean-Claude, 75116 Paris (FR); Treillet, Jean, 75116 Paris (FR); Beaumesnil, Bernard, 75116 Paris (FR)
(74) Mandataire: Ballot, Paul Denis Jacques

(57) **Abrégé**

Système d'articulation permettant d'orienter un dispositif TAC de Correction d'Atténuation de Transmission d'une caméra de scintigraphie à deux détecteurs dans deux positions successives perpendiculaires et opposées afin de permettre l'utilisation du dispositif TAC soit avec les détecteurs perpendiculaires soit avec les détecteurs parallèles. Ce système d'articulation comporte un plan de joint (9) à 45°, par rapport à l'axe longitudinal d'un support (7) de sorte que le dispositif TAC peut prendre deux positions perpendiculaires et opposées, le passage d'une position à l'autre s'obtenant par une rotation de 180° par rapport à l'axe de ce plan de joint.

## Description

La présente invention se rapporte aux caméras de scintigraphie à double détecteur à angulation variable et, plus particulièrement dans de telles caméras, une articulation permettant d'orienter un dispositif de correction d'atténuation de transmission.

Dans ce type de caméra, les détecteurs peuvent être soit perpendiculaires pour l'acquisition des images de certains organes en tomographie à 90°, soit face à face pour la tomographie à 180° ou l'acquisition de l'image du squelette sur le corps entier.

Ces caméras peuvent être équipées d'un ou deux dispositifs TAC, initiales de la traduction anglaise de Correction d'Atténuation de Transmission. Le dispositif contient une source radioactive émettrice de rayons gamma d'énergie connue qui balayent la surface active du détecteur à travers le corps du patient. On mesure, dans ce détecteur, l'atténuation d'énergie provoquée par le corps du patient. Le résultat de cette mesure est une information qui, convenablement traitée, permet de corriger l'atténuation de l'énergie des photons émis par l'organe irradié et d'obtenir ainsi une image plus précise.

La source radioactive est une tige linéaire enfermée dans une enceinte étanche au rayonnement mais pourvue d'une fente rectiligne à travers laquelle la radiation est transmise en direction du détecteur. Cette fente doit être parfaitement obturée lorsque le dispositif TAC est hors service . Cette obturation est généralement commandée mécaniquement et elle doit être totalement sécurisée. Le dispositif TAC se déplace parallèlement à lui-même dans un plan parallèle à la surface de détection du détecteur.

Dans l'état actuel de la technique, le dispositif TAC n'est utilisé que pour certaines acquisitions d'images et en particulier pour la tomographie 90°, c'est-à-dire lorsque les détecteurs sont perpendiculaires. Dans les autres cas, notamment pour l'acquisition de l'image à l'aide de deux détecteurs parallèles face à face, le dispositif TAC n'est pas orienté pour fonctionner.

La présente invention remédie à cet inconvénient en permettant l'utilisation du dispositif TAC dans les trois types de configuration mentionnés ci-dessus (tomographie 90° et 180°, corps entier).

Elle consiste en une articulation orientable constitué par un plan de joint à 45° par rapport à l'axe longitudinal du dispositif TAC de sorte qu'il peut prendre deux positions perpendiculaires et opposées et que le passage d'une position à l'autre s'obtient par une rotation de 180° par rapport à l'axe de ce plan de joint.

Les deux positions sont indexées par des pieds ou des goupilles de positionnement diamétralement opposés pénétrant dans des trous de la pièce en vis-à-vis.

L'une des deux parties de l'articulation reçoit une bague épaulée solidaire en rotation de l'autre partie et équipée de vis aptes à serrer ces deux parties l'une contre l'autre.

La transmission du mouvement de commande d'isolation de la source est assurée par un jeu de pignons situé dans la partie centrale du plan de joint.

Pendant la rotation de l'enceinte portant la source autour de l'axe du plan de joint, les pignons sont désolidarisés afin que la source reste isolée et, après repositionnement, lesdits pignons sont réengrénés en maintenant l'isolation de la source.

Le pignon menant et le pignon mené sont des pignons coniques entretoisés par deux autres pignons coniques montés sur un arbre commun situé dans l'axe du plan de joint.

Les pignons coniques sont orientés dans un sens tel que l'écartement suivant l'axe du plan de joint provoque leur désolidarisation.

L'articulation peut être soit un organe intégré dans le support, soit un organe amovible inséré entre deux parties du support, soit un organe amovible inséré entre le support et une cassette portant la source.

L'organe amovible, inséré entre le support et une cassette contenant la source, possède à une extrémité la partie mâle et à l'autre extrémité la partie femelle d'un assemblage identique à l'assemblage sécurisé de la cassette sur le support.

Les caractéristiques et avantages de l'invention apparaîtront à travers la description qui suit d'un exemple de réalisation non limitatif en référence aux dessins annexés sur lesquels:
- la figure 1 représente une vue générale d'une caméra double tête à statif ouvert, les deux détecteurs étant orientés à 90° et équipés chacun d'un dispositif TAC l'un en position de travail et l'autre en position de repos ;
- la figure 2 représente la même caméra avec les deux détecteurs face à face, l'un d'eux étant pourvu d'un dispositif TAC orienté face à l'autre détecteur ;
- la figure 3 est une vue d'un exemple de réalisation de l'articulation selon l'invention dans son orientation "d'équerre" ;
- la figure 4 est une vue de ce même exemple dans son orientation " en ligne" avant serrage des vis d'assemblage ;
- la figure 5 est une vue en coupe diamétrale de l'exemple de réalisation de l'articulation représenté figure 3, et
- la figure 6 est une vue en coupe diamétrale de ce même exemple après rotation de 180° et avant serrage des vis d'assemblage.

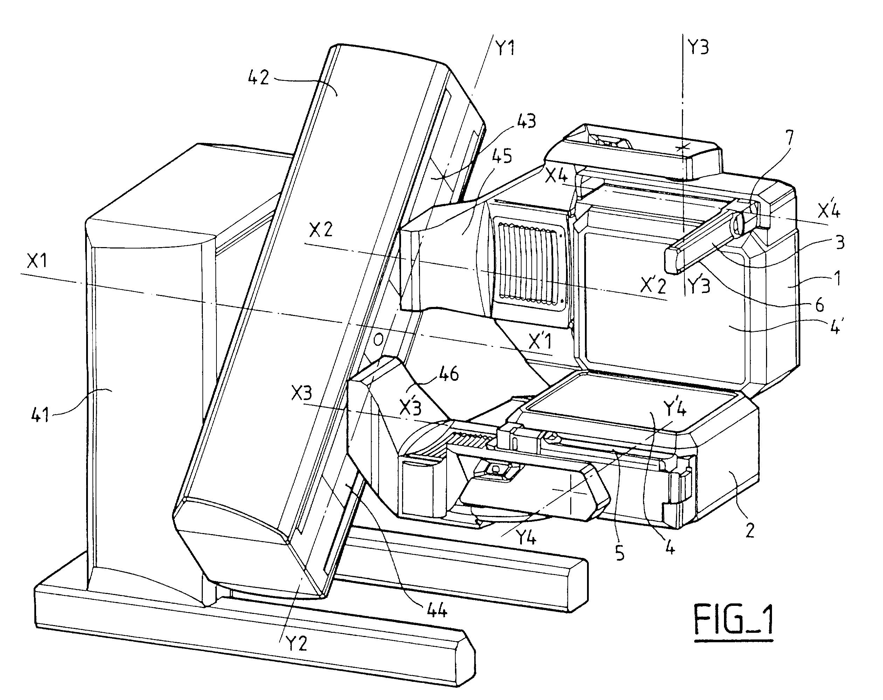

Sur la figure 1, la gamma caméra se compose d'un bâti (41) supportant une embase tournante (42) qui reçoit des chariots (43) (44) équipés respectivement de deux supports (45) (46) de détecteurs (1) (2) dont les surfaces de détection sont référencées (4)(4').
- L'embase (42) tourne autour de l'axe X1 X'1 ;
- Les chariots (43) et (44) se déplacent linéairement selon les directions OY1 OY2 ;
- Les supports de détecteurs tournent respectivement autour des axes X2 X'2 et X3 X'3 ;
- Les détecteurs tournent respectivement autour des axes Y3 Y'3 et Y4 Y'4.

On voit donc que les détecteurs peuvent prendre toute position à l'intérieur d'un certain espace et en particulier être perpendiculaires comme sur la figure ou parallèles face à face, etc.

Sur la figure 1, les deux détecteurs (1) et (2) de la caméra sont représentés perpendiculaires, un premier dispositif TAC (3) étant en position telle que sa face émettrice (6) irradie la face (4) du détecteur (2) tandis qu'un deuxième dispositif TAC (5) est en position repos. Le dispositif TAC (3) se déplace suivant l'axe X4X'4 de sorte que les rayons gamma émis balayent toute la surface (4) du détecteur (24).

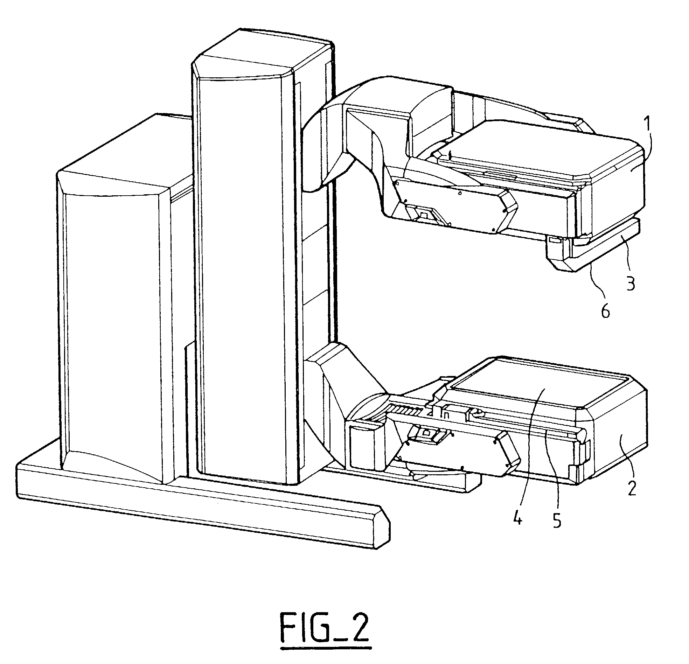

Sur la figure 2, les détecteurs (1) et (2) sont face à face, le dispositif TAC (3) est replié à 90° de sa position d'origine et a subi une rotation de 180° sur son axe afin que sa face émettrice (6) soit tournée vers la face (4) du détecteur (2). Dans cette configuration, le dispositif TAC (5)) reste en position repos.

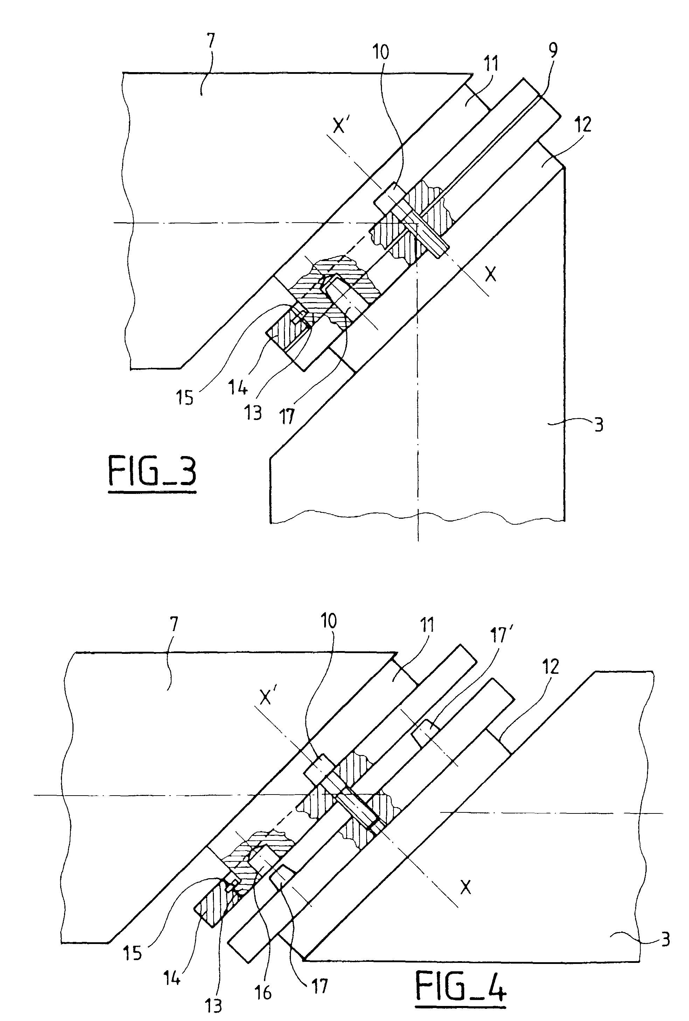

Ce double changement d'orientation correspond à une simple rotation de 180° autour de l'axe X'X (figure 3) d'un plan de joint orienté à 45° par rapport à l'axe du support (7) du dispositif TAC.

Les figures 3 et 4, qui illustrent un exemple de réalisation non limitatif, reprennent le support (7) et le dispositif TAC proprement dit (3) ; ces deux parties sont reliées par un plan de joint à 45° (9) et assemblées par deux vis (10). Les deux pièces (7) et (3) ont un profil quelconque, mais il est toujours possible d'y inscrire, dans la section à 45°, une partie cylindrique (11) (12). Le cylindre (11) se termine par un épaulement (13) entouré d'une bague (14) épaulée par un circlips (15). Dans le plan de joint (9) se trouvent au moins deux trous, dont seul le trou (16) a été représenté sur les figures 3 et 4, ces trous, diamétralement opposés, étant destinés à recevoir des pieds de positionnement (17) (17'). Pour passer de la position d'équerre (figure 3) à la position alignée (figure 4), il suffit de desserrer les vis (10) jusqu'à dégager les pieds (17)(17') de leur logement (16)(16') et de tourner le dispositif TAC (8) de 180°, ce qui entraîne la bague (14) en rotation puisqu'elle est liée au TAC (8) par les vis (10) et, éventuellement, par d'autres guides non représentés. Les pieds (17)(17') retrouvent alors le logement opposé et il ne reste plus qu'à resserrer les vis (10).

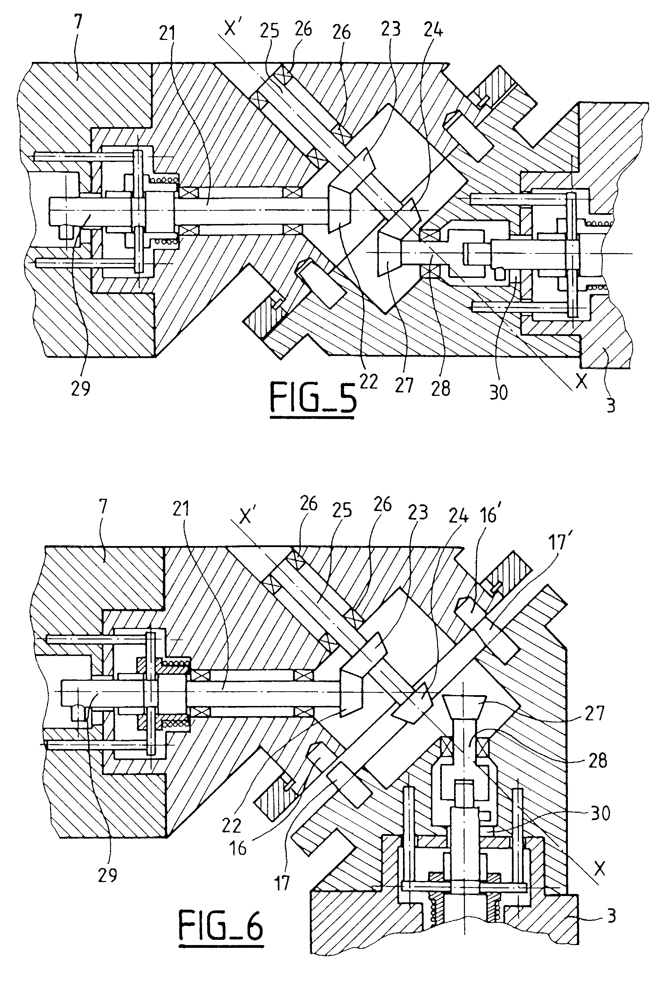

Lorsque le mécanisme d'isolation de la source se trouve sur le dispositif TAC (8), le plan de joint est simple et ne contient en son centre que des contacts pour amener le courant électrique à ce mécanisme. Si, par contre, le mécanisme est placé du côté du support (7), il faut transmettre le mouvement d'ouverture et de fermeture de la source à travers le plan de joint en assurant la position fermée, donc opaque aux radiations, pendant la rotation de 180° autour de l'axe X'X. A cet effet, on peut imaginer diverses solutions apparentées à l'exemple de réalisation non limitatif représenté sur les figures 5 et 6.

Le mécanisme d'isolation de la source transmet le mouvement par l'arbre (21) qui se termine par un pignon conique (22); celui-ci engrène sur un deuxième pignon conique (23) solidaire d'un troisième pignon conique (24) par l'intermédiaire d'un arbre (25) centré sur l'axe de rotation X'X et tournant dans un palier (26) du support (7). De l'autre côté du plan de joint, un pignon (27) solidaire d'un arbre (28) engrène sur le pignon (24) ; les arbres (21) et (28) étant parallèles et légèrement décalés (figure 5) ou perpendiculaires (figure 6).

Lorsque les vis (10) sont desserrées, les deux parties (7) et (3) s'écartent suivant l'axe X'X jusqu'au dégagement des pieds (17)(17') de leur logement (16)(16') et cela provoque l'écartement du pignon (27). Il peut être avantageux de forcer l'écartement par un dispositif à ressort qui n'est pas représenté. Ainsi, après une rotation de 180°au cours de laquelle le pignon (27) est désengagé, donc sans effet sur l'isolation de la source, ledit pignon se retrouve prêt à engrener comme représenté sur la figure 6. Les pieds (17)(17') se placent en face du logement opposé au précédent et il ne reste plus qu'à serrer les vis (10) pour rendre le dispositif TAC opérationnel à nouveau.

Le présent dispositif d'articulation peut être solidaire du support (7), du dispositif TAC (8) ou en être indépendant pour constituer une option. Cette interface comporterait alors, à une extrémité la partie mâle (29) et à l'autre la partie femelle (30) du système d'assemblage sécurisé prévu entre le support et la cassette afin qu'on puisse utiliser le dispositif TAC avec ou sans l'articulation.

Cette polyvalence d'utilisation du dispositif TAC permet d'améliorer la qualité des images pour toutes les acquisitions au bénéfice d'un meilleur diagnostic en médecine nucléaire.

## Revendications

1. Articulation orientable pour dispositif de correction d'atténuation de transmission, appelé dispositif TAC, monté sur caméra de scintigraphie double détecteur à angulation variable, recevant une enceinte de source radioactive linéaire (3) mobile en translation parallèlement à la surface de détection (4) d'un détecteur (2), caractérisée en ce qu'elle est constituée par un plan de joint (9) à 45° par rapport à l'axe longitudinal du support, de sorte que le dispositif TAC peut prendre deux positions perpendiculaires et opposées, le passage d'une position à l'autre s'obtenant par une rotation de 180° par rapport à l'axe de ce plan de joint.

2. Articulation selon la revendication 1, caractérisée en ce que les deux positions sont indexées par des pieds (17, 17') de positionnement diamétralement opposés pénétrant dans des trous de la pièce en vis-à-vis.

3. Articulation selon la revendication 1 ou 2, caractérisée en ce que l'une des deux parties de l'articulation reçoit une bague épaulée (14) solidaire en rotation de l'autre partie et équipée de vis (10) aptes à serrer ces deux parties l'une contre l'autre.

4. Articulation selon la revendication 1, 2 ou 3, caractérisée en ce que la transmission du mouvement de commande d'isolation de la source est assurée par un jeu de pignons (22) (23) (24) (27) situé dans la partie centrale du plan de joint.

5. Articulation selon la revendication 4, caractérisée en ce que, pendant la rotation de l'enceinte portant la source autour de l'axe du plan de joint, les pignons (24)(27) sont désolidarisés afin que la source reste isolée et en ce que, après repositionnement, lesdits pignons sont réengrénés en maintenant l'isolation de la source.

6. Articulation selon l'une des revendications 4 ou 5, caractérisée en ce que le pignon menant (22) et le pignon mené (27) sont des pignons coniques entretoisés par deux pignons coniques (23)(24) montés sur un arbre commun (25) situé dans l'axe du plan de joint (9).

7. Articulation selon la revendication 6, caractérisée en ce que les pignons coniques (24)(27) sont orientés dans un sens tel que l'écartement des deux parties de l'articulation suivant l'axe du plan de joint X'X provoque leur désolidarisation.

8. Articulation selon l'une quelconque des revendications précédentes, caractérisée en ce qu'elle peut être soit un organe intégré dans le support soit un organe amovible inséré entre deux parties du support soit un organe amovible inséré entre le support et une cassette portant la source.

9. Articulation selon la revendication 8, constituée par un organe amovible inséré entre le support (7) et une cassette (8) contenant la source caractérisée en ce qu'elle possède à une extrémité la partie mâle (29) et à l'autre extrémité une partie femelle (30) d'un assemblage identique à l'assemblage sécurisé de la cassette sur le support.
